# EUROPEAN PATENT SPECIFICATION

(11) **EP 4 455 656 B1**
(45) Date of publication and mention of the grant of the patent: **13.05.2026**
(21) Application number: 22910682.8
(22) Date of filing: 16.11.2022
(51) Int. Cl.: G01N 27/04, C12M 1/00, C12M 1/34, C12Q 1/02, C12M 3/00, C12M 1/42, C12M 3/06, G01N 33/483, G01N 33/487

(54) **CHANNEL DEVICE**
KANALEINRICHTUNG
DISPOSITIF DE CANAL

(30) Priority: 24.12.2021 JP 2021210941
(43) Date of publication of application: 30.10.2024
(73) Proprietor: SCREEN Holdings Co., Ltd., Kyoto-shi, Kyoto 602-8585 (JP)
(72) Inventor: NAKATANI, Noriyuki, Kyoto-shi, Kyoto 602-8585 (JP)
(74) Representative: Goddar, Heinz J.
(86) International application number: PCT/JP2022/042504
(87) International publication number: WO 2023/119963

(56) References cited:
- WO-A1-2015/181322
- WO-A1-2020/179425
- JP-A- 2020 080 697
- JP-A- 2020 137 492
- JP-A- 2020 146 015
- JP-A- 2020 146 015
- JP-A- 2021 180 631
- US-A1- 2014 065 660
- US-A1- 2021 088 468

## Description

### Technical Field

The present invention relates to a channel device for application to measurement of electrical resistance of cellular tissue.

### Background Art

Transepithelial electrical resistance (TEER) measurement has been known as a method of evaluating the barrier function of cell layers that form a membrane structure. In the TEER measurement, an insert having a tubular shape with a bottom formed by a porous membrane is placed in a recessed portion of a culture plate, and cells are cultured on the porous membrane. Working electrodes for applying current and reference electrodes for measuring a potential difference are placed inside and outside the insert. The potential difference generated between the reference electrodes is measured while the current is applied between the working electrodes, whereby electrical resistance of the cell layers is calculated.

For example, Patent Literature 1 discloses that the electrical resistance is measured by inserting electrodes (10A and 10B) from one side inside and outside a culture insertion dish (21). For the insertion of the electrodes from one side, it is however necessary to open the top of the culture insertion dish (21). This technique is not applicable to a device which does not have such an opening at the top. JP2020146015A also discloses a channel device for application to measurement of electrical resistance of cellular tissue.

On the other hand, Non Patent Literature 1 discloses a channel device in which electrodes are placed on a lid which closes a top opening of a culture vessel. Placing the electrodes on the lid in this manner allows the measurement of the electrical resistance of cell layers being cultured in a measurement chamber.

### Citation List

### Patent Literature

Patent Literature 1: Japanese Patent Application Laid-Open No. 2005-137307

### Non Patent Literature

Non Patent Literature 1: Booth R, Kim H. Characterization of a microfluidic in vitro model of the blood-brain barrier (mu BBB) Lab Chip. 2012 Apr. 24;12(10):1784-92.

### Summary of Invention

### Technical Problem

During the assembly of the channel device as disclosed in Non Patent Literature 1, the electrodes are misaligned relative to the measurement chamber if the lid is mounted in a position misaligned relative to the measurement chamber. Also, when the electrodes are positioned near an edge of the measurement chamber, there have been cases in which the misalignment of the lid fluctuates the area (contact area) of the electrodes in contact with a liquid in the measurement chamber. Thus, the misalignment of the electrodes or the fluctuations in contact area causes fluctuations in current density distribution in the measurement chamber. This results in a danger that measurement results of the electrical resistance fluctuate.

It is an object of the present invention to provide a technique capable of reducing variations in measurement results of electrical resistance due to the assembly accuracy of a channel device.

### Solution to Problem

To solve the aforementioned problem, a first aspect is intended for a channel device for application to measurement of electrical resistance of cellular tissue, comprising: an intermediate member including a measurement chamber formed by a through hole passing therethrough in a first direction; a first lid member positioned on a surface of the intermediate member which is on a first side as seen in the first direction and closing an opening at a first edge of the measurement chamber as seen in the first direction; a second lid member positioned on a surface of the intermediate member which is on a second side as seen in the first direction and closing an opening at a second edge of the measurement chamber as seen in the first direction; a porous membrane positioned in the measurement chamber and partitioning the measurement chamber into a first chamber on the first side and a second chamber on the second side as seen in the first direction, the porous membrane being permeable to liquid; a first-side working electrode and a first-side reference electrode both positioned on a surface of the first lid member which is on the second side as seen in the first direction and overlapping the measurement chamber as seen in the first direction; and a second-side working electrode and a second-side reference electrode both positioned on a surface of the second lid member which is on the first side as seen in the first direction and overlapping the measurement chamber as seen in the first direction, wherein the first-side working electrode includes a first first-side working portion, and a second first-side working portion spaced toward a first side in a second direction intersecting the first direction apart from the first first-side working portion, wherein a dimension from an edge of the first first-side working portion which is on a second side as seen in the second direction to an edge of the second first-side working portion which is on the first side as seen in the second direction is greater than the width of the measurement chamber as measured in the second direction, and wherein a distance between the first first-side working portion and the second first-side working portion as measured in the second direction is smaller than the width of the measurement chamber.

A second aspect is intended for the channel device of the first aspect, wherein the first-side reference electrode includes a first first-side reference portion, and a second first-side reference portion spaced toward the first side in the second direction apart from the first first-side reference portion, and wherein the first first-side reference portion and the second first-side reference portion are positioned between the first first-side working portion and the second first-side working portion as seen in the second direction.

A third aspect is intended for the channel device of the second aspect, wherein the first-side working electrode further includes a third first-side working portion positioned between the first first-side reference portion and the second first-side reference portion as seen in the second direction.

A fourth aspect is intended for the channel device of the third aspect, wherein the width of the first first-side working portion as measured in the second direction is greater than the width of the third first-side working portion as measured in the second direction.

A fifth aspect is intended for the channel device of the third or fourth aspect, wherein the first-side working electrode further includes a fourth first-side working portion positioned between the first first-side reference portion and the second first-side reference portion as seen in the second direction and spaced toward the first side in the second direction apart from the third first-side working portion.

A sixth aspect is intended for the channel device of the fifth aspect, wherein the second-side working electrode includes a first second-side working portion, and a second second-side working portion spaced toward the first side in the second direction apart from the first second-side working portion, wherein the first second-side working portion is in opposed relation to the first first-side working portion as seen in the first direction, wherein the second second-side working portion is in opposed relation to the second first-side working portion as seen in the first direction, wherein a dimension from an edge of the first second-side working portion which is on the second side as seen in the second direction to an edge of the second second-side working portion which is on the first side as seen in the second direction is greater than the width of the measurement chamber as measured in the second direction, and wherein a distance between the first second-side working portion and the second second-side working portion as measured in the second direction is smaller than the width of the measurement chamber as measured in the second direction.

A seventh aspect is intended for the channel device of the sixth aspect, wherein the second-side reference electrode includes a first second-side reference portion, and a second second-side reference portion spaced toward the first side in the second direction apart from the first second-side reference portion, and wherein the first second-side reference portion and the second second-side reference portion are positioned between the first second-side working portion and the second second-side working portion as seen in the second direction.

An eighth aspect is intended for the channel device of the seventh aspect, wherein the second-side working electrode further includes a third second-side working portion positioned between the first second-side reference portion and the second second-side reference portion as seen in the second direction.

A ninth aspect is intended for the channel device of the eighth aspect, wherein the second-side working electrode further includes a fourth second-side working portion positioned between the first second-side reference portion and the second second-side reference portion as seen in the second direction and spaced toward the first side in the second direction apart from the third second-side working portion.

A tenth aspect is intended for the channel device of any one of the first to ninth aspects, wherein the intermediate member includes a first intermediate member, and a second intermediate member positioned on the first side of the first intermediate member as seen in the first direction, wherein the first intermediate member includes the first chamber, wherein the second intermediate member includes the second chamber, and wherein the porous membrane is positioned between the first intermediate member and the second intermediate member as seen in the first direction.

An eleventh aspect is intended for the channel device of any one of the first to tenth aspects, wherein the intermediate member includes a first channel in communication with the first chamber, and a second channel in communication with the second chamber.

A twelfth aspect is intended for the channel device of the eleventh aspect, wherein the first lid member includes a first through hole in communication with the first channel, and a second through hole in communication with the second channel.

A thirteenth aspect is intended for the channel device of any one of the first to twelfth aspects, wherein the measurement chamber, the first first-side working portion, and the second first-side working portion extend in a third direction intersecting the first and second directions.

A fourteenth aspect is intended for the channel device of the thirteenth aspect, wherein the first first-side working portion and the second first-side working portion extend from a position spaced toward a first side in the third direction apart from the measurement chamber to a position spaced toward a second side in the third direction apart from the measurement chamber.

### Advantageous Effects of Invention

According to the channel device of the first to fourteenth aspects, even if the first lid member is displaced in the second direction with respect to the measurement chamber, the first first-side working portion and the second first-side working portion are disposed at both edges as seen in the second direction in the measurement chamber. This allows good voltage application at both edges of the measurement chamber even if the first lid member is displaced. Even if the first lid member is displaced, fluctuations in contact area where the first-side working electrode contacts the liquid in the measurement chamber are suppressed. This reduces variations in measurement results of electrical resistance due to the mounting accuracy of the first lid member.

According to the channel device of the third aspect, good voltage application near the center of the measurement chamber as seen in the second direction is allowed by providing the third first-side working portion.

According to the channel device of the fourth aspect, the allowable range of displacement of the first lid member with respect to the measurement chamber is increased by increasing the first first-side working portion.

According to the channel device of the fifth aspect, good voltage application near the center of the measurement chamber as seen in the second direction is allowed by providing the fourth first-side working portion.

According to the channel device of the sixth aspect, the second-side electrode {second-side working electrode} is placed in opposed relation to the first-side electrode {first-side working electrode}, whereby a uniform voltage is applied to the cellular tissue lying between the electrodes.

According to the channel device of the eighth aspect, good voltage application near the center of the measurement chamber as seen in the second direction is allowed by providing the third second-side working portion.

According to the channel device of the ninth aspect, good voltage application near the center of the measurement chamber as seen in the second direction is allowed by providing the fourth second-side working portion.

According to the channel device of the eleventh aspect, liquid is exchangeable between the first and second chambers.

According to the channel device of the twelfth aspect, liquid can be supplied through the first through hole of the first lid member into the measurement chamber. The liquid in the measurement chamber can be discharged through the second through hole.

According to the channel device of the fourteenth aspect, the first first-side working portion and the second first-side working portion traverse the measurement chamber in the third direction. Thus, voltage is applied to the measurement chamber throughout the third direction even if the first lid member is displaced in the third direction with respect to the measurement chamber. This reduces variations in measurement results of electrical resistance due to the assembly accuracy of the channel device.

### Brief Description of Drawings

[fig. 1] Fig. 1 is a sectional view of a channel device according to an embodiment.
[fig. 2] Fig. 2 is an exploded sectional view of the channel device according to the embodiment.
[fig. 3] Fig. 3 is a top plan view of an upper working electrode and an upper reference electrode.
[fig. 4] Fig. 4 is a sectional view of the channel device according to the embodiment.
[fig. 5] Fig. 5 is a diagram showing a circuit for measuring electrical resistance of cellular tissue.
[fig. 6] Fig. 6 is a view showing sectional structures of channel devices used for simulation.
[fig. 7] Fig. 7 is a graph showing simulation results of a current density distribution.
[fig. 8] Fig. 8 is a graph showing frequency characteristics of the channel device according to the embodiment and a channel device according to a comparative example.

### Description of Embodiments

An embodiment according to the present invention will now be described with reference to the drawings. Components described in the embodiment are merely illustrative, and there is no intention to limit the scope of the present invention thereto. In the drawings, the dimensions of components and the number of components are shown in exaggeration or in simplified form, as appropriate, for the sake of easier understanding in some cases.

### <1. Embodiment>

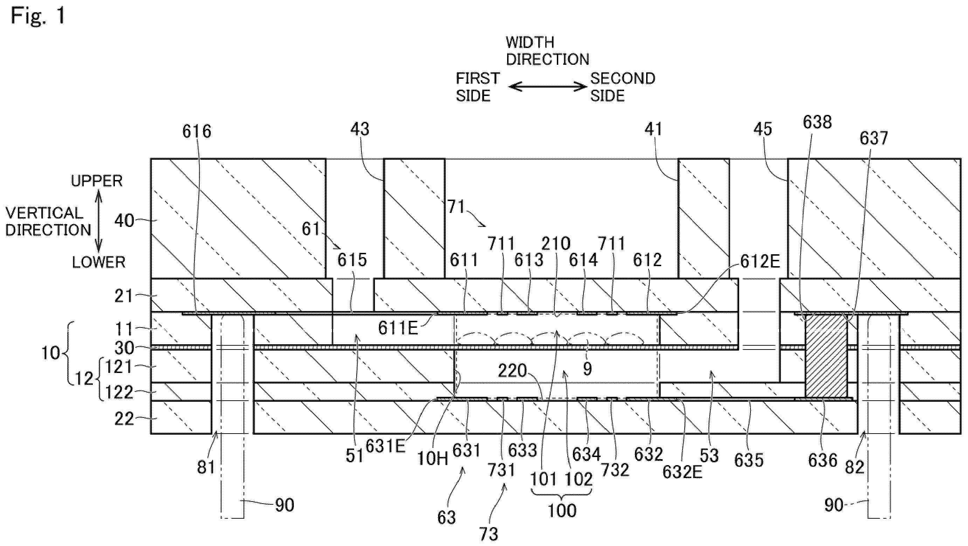

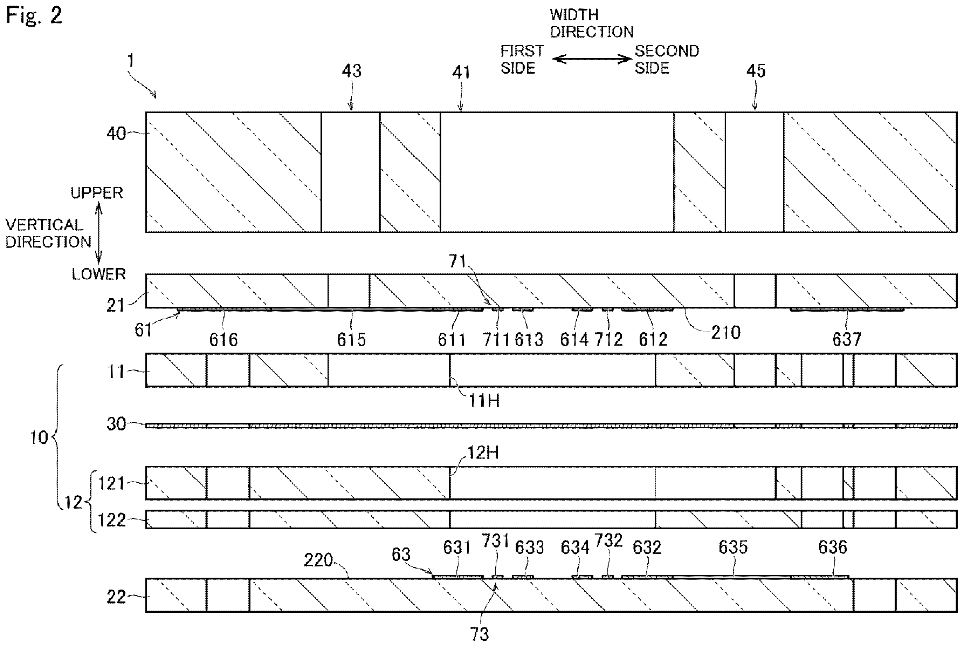

Fig. 1 is a sectional view of a channel device 1 according to an embodiment. Fig. 2 is an exploded sectional view of the channel device 1 according to the embodiment. The channel device 1 is what is called a microchannel device in which a measurement chamber 100 that is an internal space is a closed space with the exceptions of a fine first channel 51 and a fine second channel 53. The channel device 1 is applicable to a device for measuring electrical resistance (resistance, reactance, or impedance) of cellular tissue 9 cultured in the measurement chamber 100 by means of a four-terminal measurement method.

As shown in Figs. 1 and 2, the channel device 1 includes an intermediate member 10, a first lid member 21, and a second lid member 22. The intermediate member 10, the first lid member 21, and the second lid member 22 are planar. In the channel device 1, the intermediate member 10 is disposed on an upper surface 220 of the second lid member 22. The first lid member 21 is disposed on an upper surface of the intermediate member 10.

As shown in Fig. 1, the intermediate member 10 includes the measurement chamber 100 therein. The measurement chamber 100 forms a space that can accommodate liquid such as a culture solution. As shown in Figs. 1 and 2, the intermediate member 10 includes a first intermediate member 11 and a second intermediate member 12. The second intermediate member 12 includes an upper member 121 and a lower member 122. The upper member 121 is disposed on an upper surface of the lower member 122. The first intermediate member 11 is positioned above the second intermediate member 12.

The intermediate member 10 is made of PET (polyethylene terephthalate), for example. The first intermediate member 11 is preferably permeable to light, and more preferably colorless and transparent. As shown in Fig. 1, the intermediate member 10 has a through hole 10H vertically passing through the intermediate member 10. An inner surface of the through hole 10H forms an inner side surface of the measurement chamber 100.

The first lid member 21 and the second lid member 22 are transparent substrates made of quartz glass, for example. As shown in Fig. 1, the first lid member 21 closes an upper opening of the measurement chamber 100. That is, an upper portion of the measurement chamber 100 is blocked by the first lid member 21. The second lid member 22 closes a lower opening of the measurement chamber 100. That is, a lower portion of the measurement chamber 100 is blocked by the second lid member 22.

As shown in Figs. 1 and 2, the channel device 1 includes a porous membrane 30. The porous membrane 30 is a sheet-like permeable membrane that is permeable to liquid. The porous membrane 30 is made of PC (polycarbonate), PTFE (polytetrafluoroethylene), or PET, for example. The porous membrane 30 is preferably permeable to light. The porous membrane 30 is disposed between the first intermediate member 11 and the second intermediate member 12 as seen in a vertical direction.

The porous membrane 30 partitions the measurement chamber 100 (the through hole 10H) into an upper first chamber 101 and a lower second chamber 102. As shown in Fig. 1, the cellular tissue 9 is disposed in the first chamber 101 of the measurement chamber 100 by being supported on an upper surface of the porous membrane 30 in the measurement chamber 100. An upper surface of part of the porous membrane 30 which is disposed in the measurement chamber 100 (the through hole 10H) may be coated with a cell adhesion factor (such as collagen).

As shown in Fig. 1, the first chamber 101 of the measurement chamber 100 is defined by an inner surface of a first through hole 11H which vertically passes through the first intermediate member 11, the upper surface of the porous membrane 30, and a lower surface 210 of the first lid member 21. The second chamber 102 of the measurement chamber 100 is defined by an inner surface of a second through hole 12H which vertically passes through the second intermediate member 12, a lower surface of the porous membrane 30, and the upper surface 220 of the second lid member 22.

As shown in Figs. 1 and 2, the channel device 1 includes a planar top member 40. The top member 40 is disposed on an upper surface of the first lid member 21. The top member 40 is made of PET, for example. The top member 40 has a viewing opening 41. The viewing opening 41 is a though hole which vertically passes through the top member 40. The viewing opening 41 is disposed immediately over the measurement chamber 100. In other words, the viewing opening 41 vertically overlies the measurement chamber 100.

As shown in Fig. 1, the intermediate member 10 includes the first channel 51. The first channel 51 is in communication with the first chamber 101 of the measurement chamber 100. The term "in communication" refers to a state of being coupled so that a fluid can pass through. The first channel 51 is a channel for supplying liquid such as a culture solution to the first chamber 101 of the measurement chamber 100. The first channel 51 is in the shape of a tube vertically passing through the first intermediate member 11 and surrounded by an inner surface of a through hole extending in a width direction (a second direction), an upper surface of the upper member 121 of the second intermediate member 12, and the lower surface 210 of the first lid member 21. The width direction is a direction intersecting the vertical direction. Preferably, the width direction is a direction orthogonal to the vertical direction.

As shown in Fig. 1, the intermediate member 10 includes the second channel 53. The second channel 53 is in communication with the second chamber 102 of the measurement chamber 100. The second channel 53 is a channel for discharging liquid from the second chamber 102 of the measurement chamber 100. The second channel 53 is in the shape of a tube vertically passing through the upper member 121 of the second intermediate member 12 and surrounded by an inner surface of a through hole extending in the width direction, a lower surface of the first intermediate member 11, and the upper surface of the lower member 122 of the second intermediate member 12.

As shown in Fig. 1, part of an upper edge of the first channel 51 is in communication with a first through hole 43 which vertically passes through the top member 40 and the first lid member 21. Also, part of an upper edge of the second channel 53 is in communication with a second through hole 45 which vertically passes through the top member 40 and the first lid member 21. Liquid can be supplied through the first through hole 43 into the measurement chamber 100. The liquid in the measurement chamber 100 can be discharged through the second through hole 45.

### <Electrodes>

As shown in Figs. 1 and 2, the channel device 1 includes an upper working electrode 61, a lower working electrode 63, an upper reference electrode 71, and a lower reference electrode 73. The upper working electrode 61 and the upper reference electrode 71 are disposed on the lower surface 210 of the first lid member 21. The lower working electrode 63 and the lower reference electrode 73 ae disposed on the upper surface 220 of the second lid member 22. Each of the electrodes is formed by vacuum evaporation or the like. Part of each of the electrodes (e.g., a portion facing the inside of the measurement chamber 100) may be covered with an insulation film such as a silicon oxide film.

The upper working electrode 61, the lower working electrode 63, the upper reference electrode 71, and the lower reference electrode 73 have portions vertically overlapping the measurement chamber 100. That is, the upper working electrode 61, the lower working electrode 63, the upper reference electrode 71, and the lower reference electrode 73 have portions vertically overlapping the measurement chamber 100.

### <Upper Working Electrode 61>

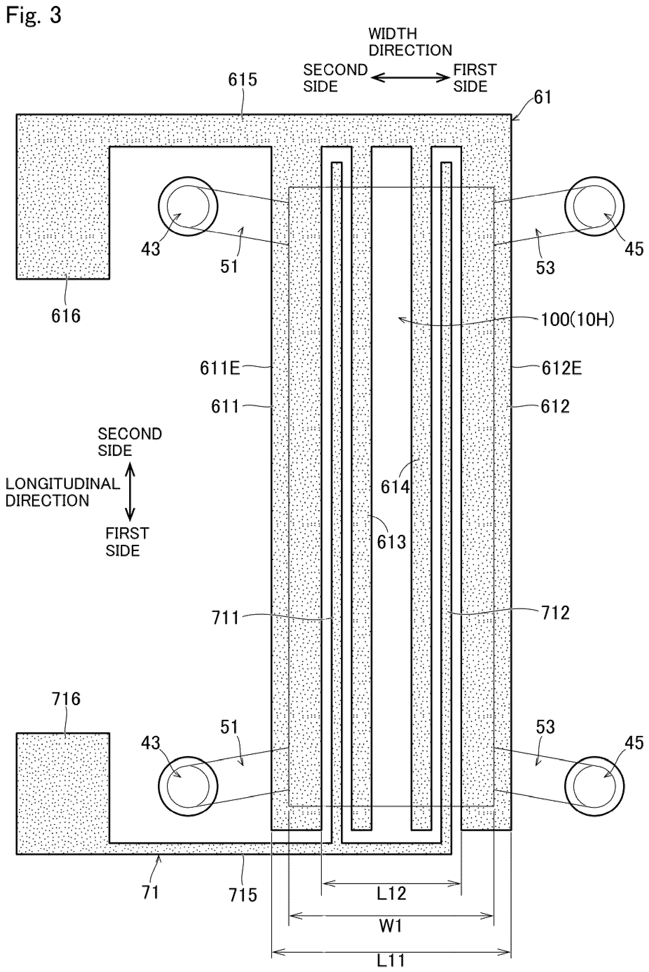

Fig. 3 is a top plan view of the upper working electrode 61 and the upper reference electrode 71. As shown in Fig. 3, the upper working electrode 61 includes a first upper working portion 611, a second upper working portion 612, a third upper working portion 613, and a fourth upper working portion 614.

As shown in Fig. 3, the measurement chamber 100 extends in a longitudinal direction (a third direction). The longitudinal direction is a direction intersecting the vertical direction and the width direction. Preferably, the longitudinal direction is a direction orthogonal to the vertical direction and the width direction. The first upper working portion 611, the second upper working portion 612, the third upper working portion 613, and the fourth upper working portion 614 also extend linearly in the longitudinal direction.

The first upper working portion 611, the second upper working portion 612, the third upper working portion 613, and the fourth upper working portion 614 overlap the measurement chamber 100. As shown in Fig. 3, the dimensions of the first upper working portion 611, the second upper working portion 612, the third upper working portion 613, and the fourth upper working portion 614 as measured in the longitudinal direction are greater than the dimension of the measurement chamber 100 as measured in the longitudinal direction. The first upper working portion 611, the second upper working portion 612, the third upper working portion 613, and the fourth upper working portion 614 extend from a position spaced toward a first side in the longitudinal direction apart from the measurement chamber 100 to a position spaced toward a second side in the longitudinal direction apart from the measurement chamber 100. In other words, the first upper working portion 611, the second upper working portion 612, the third upper working portion 613, and the fourth upper working portion 614 traverse the measurement chamber 100 in the longitudinal direction.

As shown in Figs. 1 and 3, the upper working electrode 61 includes a wiring portion 615 and a pad portion 616. The wiring portion 615 is spaced toward the second side in the longitudinal direction apart from the measurement chamber 100. The wiring portion 615 extends in the width direction and is connected to the pad portion 616. Respective second end portions of the first, second, third, and fourth upper working portions 611, 612, 613, and 614 as seen in the longitudinal direction are connected to the wiring portion 615. That is, the first upper working portion 611, the second upper working portion 612, the third upper working portion 613, and the fourth upper working portion 614 are electrically connected to the pad portion 616 through the wiring portion 615.

As shown in Figs. 1 and 3, the second upper working portion 612 is spaced toward a first side in the width direction apart from the first upper working portion 611. The third upper working portion 613 is spaced toward the first side in the width direction apart from the first upper working portion 611. The fourth upper working portion 614 is spaced toward a second side in the width direction apart from the second upper working portion 612. The fourth upper working portion 614 is spaced toward the first side in the width direction apart from the third upper working portion 613.

### <Upper Reference Electrode 71>

The upper reference electrode 71 includes a first upper reference portion 711 and a second upper reference portion 712. The first upper reference portion 711 and the second upper reference portion 712 extend linearly in the longitudinal direction.

As shown in Fig. 3, the first upper reference portion 711 and the second upper reference portion 712 extend from a position spaced toward the first side in the longitudinal direction apart from the measurement chamber 100 to a position spaced toward the second side in the longitudinal direction apart from the measurement chamber 100. In other words, the first upper reference portion 711 and the second upper reference portion 712 traverse the measurement chamber 100 in the longitudinal direction.

As shown in Figs. 1 and 3, the second upper reference portion 712 is spaced toward the first side in the width direction apart from the first upper reference portion 711. The first upper reference portion 711 is spaced toward the first side in the width direction apart from the first upper working portion 611. The second upper reference portion 712 is spaced toward the second side in the width direction apart from the second upper working portion 612.

The first upper reference portion 711 is disposed between the first upper working portion 611 and the third upper working portion 613 as seen in the width direction. The second upper reference portion 712 is disposed between the second upper working portion 612 and the fourth upper working portion 614 as seen in the width direction.

The third upper working portion 613 and the fourth upper working portion 614 are disposed between the first upper reference portion 711 and the second upper reference portion 712 as seen in the width direction.

As shown in Fig. 3, the upper reference electrode 71 includes a wiring portion 715 and a pad portion 716. The wiring portion 715 is spaced toward the first side in the longitudinal direction apart from the measurement chamber 100. The wiring portion 715 extends in the width direction and is connected to the pad portion 716. As shown in Fig. 3, respective first end portions of the first and second upper reference portions 711 and 712 as seen in the longitudinal direction are connected to the wiring portion 715. The respective first end portions of the first and second upper reference portions 711 and 712 as seen in the longitudinal direction are connected to the wiring portion 715. That is, the first upper reference portion 711 and the second upper reference portion 712 are electrically connected to the pad portion 716 through the wiring portion 715.

### <Lower Working Electrode 63>

As shown in Fig. 1, the lower working electrode 63 includes a first lower working portion 631, a second lower working portion 632, a third lower working portion 633, and a fourth lower working portion 634.

The first lower working portion 631, the second lower working portion 632, the third lower working portion 633, and the fourth lower working portion 634 are identical in size and shape with the first upper working portion 611, the second upper working portion 612, the third upper working portion 613, and the fourth upper working portion 614, respectively.

The first, second, third, and fourth upper working portions 611, 612, 613, and 614 of the upper working electrode 61 are in symmetric relation to the first, second, third, and fourth lower working portions 631, 632, 633, and 634 of the lower working electrode 63 with respect to a predetermined plane and with respect to a plane orthogonal to the vertical direction.

The first lower working portion 631, the second lower working portion 632, the third lower working portion 633, and the fourth lower working portion 634 are in opposed relation to the first upper working portion 611, the second upper working portion 612, the third upper working portion 613, and the fourth upper working portion 614, respectively, as seen in the vertical direction. In this manner, the upper working electrode 61 and the lower working electrode 63 are placed in opposed relation to each other, whereby a uniform voltage is applied to the cellular tissue 9 lying between the electrodes.

Although not shown in detail, the first lower working portion 631, the second lower working portion 632, the third lower working portion 633, and the fourth lower working portion 634 traverse the measurement chamber 100 in the longitudinal direction.

The second lower working portion 632 is spaced toward the first side in the width direction apart from the first lower working portion 631. The third lower working portion 633 is spaced toward the first side in the width direction apart from the first lower working portion 631. The fourth lower working portion 634 is spaced toward the second side in the width direction apart from the second lower working portion 632. The fourth lower working portion 634 is spaced toward the first side in the width direction apart from the third lower working portion 633.

As shown in Fig. 1, the lower working electrode 63 includes a wiring portion 635 and a first pad portion 636. The first lower working portion 631, the second lower working portion 632, the third lower working portion 633, and the fourth lower working portion 634 are connected to the wiring portion 635. The wiring portion 635 is connected to the first pad portion 636. A second pad portion 637 is a conductive film provided on the lower surface 210 of the first lid member 21. As shown in Fig. 1, the first pad portion 636 and the second pad portion 637 are electrically connected to each other through a conductive member 638. The conductive member 638 is inserted in a through hole which passes through the first intermediate member 11, the second intermediate member 12, and the porous membrane 30.

### <Lower Reference Electrode 73>

The lower reference electrode 73 includes a first lower reference portion 731 and a second lower reference portion 732. The first lower reference portion 731 and the second lower reference portion 732 extend linearly in the longitudinal direction. Although not shown in detail, the first lower reference portion 731 and the second lower reference portion 732 traverse the measurement chamber 100 in the longitudinal direction.

The first lower reference portion 731 and the second lower reference portion 732 are identical in size and shape with the first upper reference portion 711 and the second lower reference portion 732, respectively. The first and second upper reference portions 711 and 712 are in symmetric relation to the first and second lower reference portions 731 and 732 with respect to a plane orthogonal to the vertical direction.

The first and second lower reference portions 731 and 732 are in opposed relation to the first and second upper reference portions 711 and 712, respectively, of the upper reference electrode 71 as seen in the vertical direction.

The second lower reference portion 732 is spaced toward the first side in the width direction apart from the first lower reference portion 731. The first and second lower reference portions 731 and 732 are disposed between the first and third lower working portions 631 and 633 of the lower working electrode 63 as seen in the width direction.

The third lower working portion 633 and the fourth lower working portion 634 are disposed between the first lower reference portion 731 and the second lower reference portion 732 as seen in the width direction.

Like the upper reference electrode 71, the lower reference electrode 73 includes a wiring portion and a pad portion. The first lower reference portion 731 and the second lower reference portion 732 are electrically connectable to measurement equipment outside the measurement chamber 100 through the wiring portion and the pad portion.

As shown in Fig. 1, the channel device 1 includes conduction holes 81 and 82. Each of the conduction holes 81 and 82 is defined by an inner surface of a through hole which vertically passes through the intermediate member 10, the porous membrane 30, and the second lid member 22, and the lower surface 210 of the first lid member 21 which closes an upper portion of the through hole. The conduction hole 81 is spaced toward the second side in the width direction apart from the measurement chamber 100. The conduction hole 82 is spaced toward the first side in the width direction apart from the measurement chamber 100. As shown in Fig. 1, each of the conduction holes 81 and 82 allows a conductive probe pin 90 (external electrode) to be inserted therein.

As shown in Fig. 1, the pad portion 616 of the upper working electrode 61 is disposed in the conduction hole 81. Also, the second pad portion 637 is disposed in the conduction hole 82. The upper working electrode 61 is electrically continuous with the probe pin 90 through the pad portion 616 in the conduction hole 81. The lower working electrode 63 is electrically continuous with the probe pin 90 through the second pad portion 637 in the conduction hole 82. Although not shown, the channel device 1 is provided with conduction holes for making the upper reference electrode 71 and the lower reference electrode 73 electrically continuous with respective external electrodes.

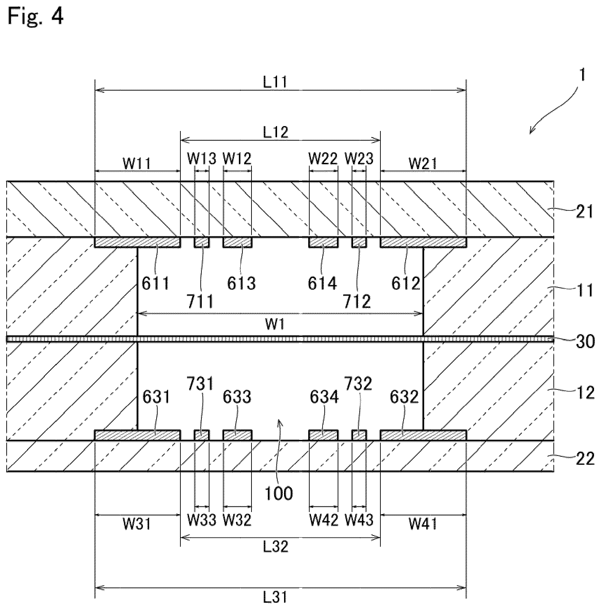

Fig. 4 is a sectional view of the channel device 1 according to the embodiment. As shown in Figs. 3 and 4, a dimension L11 from a second edge 611E of the first upper **working** portion 611 as seen in the width direction to a first edge 612E of the second upper working portion 612 as seen in the width direction is greater than the width W1 of the measurement chamber 100 as measured in the width direction (L11 > W1). A distance L12 between the first upper working portion 611 and the second upper working portion 612 as measured in the width direction is smaller than the width W1 of the measurement chamber 100 (L12 < W1).

By satisfying L11 > W1 > L12, the first upper working portion 611 and the second upper working portion 612 are disposed across the inside and outside of the measurement chamber 100 at the edges of the measurement chamber 100 as seen in the width direction. Thus, fluctuations in total area of the parts of the first and second upper working portions 611 and 612 which are disposed within the measurement chamber 100 are suppressed even if the first lid member 21 is displaced in the width direction with respect to the measurement chamber 100. In other words, fluctuations in contact area where the first upper working portion 611 and the second upper working portion 612 contact the liquid in the measurement chamber 100 are suppressed. This reduces variations in measurement results of electrical resistance due to the assembly accuracy of the channel device 1 (the mounting accuracy of the first lid member 21).

Even if the first lid member 21 is displaced in the width direction with respect to the measurement chamber 100, the first upper working portion 611 and the second upper working portion 612 are disposed at both edges as seen in the width direction in the first chamber 101 of the measurement chamber 100. This allows good voltage application near both edges as seen in the width direction in the measurement chamber 100 even if the first lid member 21 is displaced. Thus, variations in measurement results of electrical resistance due to the assembly accuracy of the channel device 1 are reduced.

Since the first and second upper working portions 611 and 612 of the upper **working** electrode 61 traverse the measurement chamber 100 in the longitudinal direction, voltage is applied throughout the length of the measurement chamber 100 even if the first lid member 21 is displaced in the longitudinal direction. This suppresses variations in measurement performance among multiple channel devices 1. Also, since the first and second lower working portions 631 and 632 of the lower working electrode 63 traverse the measurement chamber 100 in the longitudinal direction, voltage is applied throughout the length of the measurement chamber 100 even if the second lid member 22 is displaced in the longitudinal direction. Thus, variations in measurement results of electrical resistance due to the assembly accuracy of the channel device 1 are reduced.

As shown in Fig. 4, a dimension L31 from a second edge 631E of the first lower working portion 631 as seen in the width direction to a first edge 632E of the second lower working portion 632 as seen in the width direction is greater than the width W1 of the measurement chamber 100 (L31 > W1). A distance L32 between the first lower working portion 631 and the second lower working portion 632 as measured in the width direction is smaller than the width W1 of the measurement chamber 100 (L32 < W1).

By satisfying L31 > W1 > L32, fluctuations in total area of the parts of the first and second lower working portions 631 and 632 which are disposed within the measurement chamber 100 are suppressed even if the first lid member 21 is displaced in the width direction with respect to the measurement chamber 100 (the through hole 10H). In other words, fluctuations in contact area where the first lower working portion 631 and the second lower working portion 632 contact the liquid in the measurement chamber 100 are suppressed. This suppresses fluctuations in conditions under which voltage is applied to the liquid in the measurement chamber 100. Thus, variations in measurement results of electrical resistance due to the assembly accuracy of the channel device 1 (the mounting accuracy of the first lid member 21) are reduced.

Even if the second lid member 22 is displaced in the width direction with respect to the measurement chamber 100, the first lower working portion 631 and the second lower working portion 632 are disposed at both edges as seen in the width direction in the second chamber 102 of the measurement chamber 100. This allows good voltage application near both edges as seen in the width direction in the measurement chamber 100. Thus, variations in measurement results of electrical resistance due to the assembly accuracy of the channel device 1 are reduced.

As shown in Fig. 4, the width W11 of the first upper working portion 611 as measured in the width direction is preferably greater than the width W12 of the third upper working portion 613 as measured in the width direction. The width W11 and the width W12 are preferably greater than the width W13 of the first upper reference portion 711 as measured in the width direction (W11 > W12 > W13). Thus, the allowable range of displacement of the first lid member 21 with respect to the measurement chamber 100 is increased by increasing the width W11 of the first upper working portion 611.

The width W21 of the second upper working portion 612 is preferably equal to the width W11 of the first upper working portion 611. The width W22 of the fourth upper working portion 614 is preferably equal to the width W12 of the third upper working portion 613. The width W23 of the second upper reference portion 712 is preferably equal to the width W13 of the first upper reference portion 711.

The width W31 of the first lower working portion 631 as measured in the width direction is preferably greater than the width W32 of the third lower working portion 633 as measured in the width direction. The width W31 and the width W32 are preferably greater than the width W33 of the first lower reference portion 731 as measured in the width direction (W31 > W32 > W33). Thus, the allowable range of displacement of the second lid member 22 is increased by increasing the width W31 of the first lower working portion 631.

The width W41 of the second lower working portion 632 is preferably equal to the width W31 of the first lower working portion 631. The width W42 of the fourth lower working portion 634 is preferably equal to the width W32 of the third lower working portion 633. The width W43 of the second lower reference portion 732 is preferably equal to the width W33 of the first lower reference portion 731.

The upper working electrode 61 includes the third upper working portion 613 and the fourth upper working portion 614, thereby allowing good voltage application near the center of the first chamber 101 as seen in the width direction in the measurement chamber 100. The lower working electrode 63 includes the third lower working portion 633 and the fourth lower working portion 634, thereby allowing good voltage application near the center of the second chamber 102 as seen in the width direction in the measurement chamber 100.

### <Electrical Resistance Measurement>

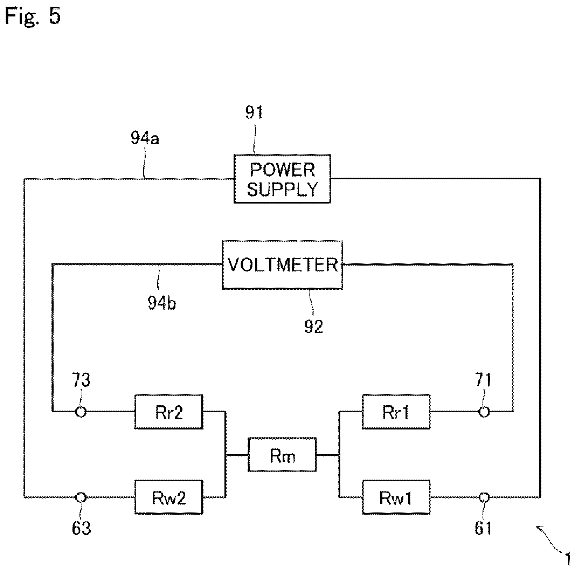

Fig. 5 is a diagram showing a circuit for measuring the electrical resistance of the cellular tissue 9. For the measurement of the electrical resistance of the cellular tissue 9, a power supply device 91 and a voltmeter 92 are connected to the channel device 1. Specifically, the power supply device 91 has an output terminal electrically connected through a conductor 94a to the upper working electrode 61 and the lower working electrode 63. The voltmeter 92 has an input terminal electrically connected through a conductor 94b to the upper reference electrode 71 and the lower reference electrode 73. The conductors 94a and 94b are connected to the electrodes through the probe pins 90.

The cellular tissue 9 is held on the upper surface of the porous membrane 30 in the measurement chamber 100. A supplying tube is connected to the first through hole 43 of the top member 40 to supply liquid (such as a culture solution) into the measurement chamber 100. The liquid is supplied through the supplying tube to the first channel 51 and the measurement chamber 100. A discharging tube is connected to the second through hole 45 of the top member 40. The liquid is discharged from the measurement chamber 100 and the second channel 53 through the discharging tube. As a result, the liquid is exchanged (or circulated) in the first and second chambers 101 and 102 of the measurement chamber 100, as appropriate.

With reference to Fig. 5, a resistance Rm corresponds to the electrical resistance of a portion of the porous membrane 30 which is disposed in the measurement chamber 100 and the cellular tissue 9 supported by the porous membrane 30 (which are referred to hereinafter as "cellular portions"). A resistance Rw1 corresponds to the electrical resistance of the liquid between the upper working electrode 61 and the cellular portions (specifically, the liquid in the first chamber 101 of the measurement chamber 100). A resistance Rw2 corresponds to the electrical resistance of the liquid between the lower working electrode 63 and the cellular portions (specifically, the liquid in the second chamber 102).

With reference to Fig. 5, a resistance Rr1 corresponds to the electrical resistance of the liquid between the upper reference electrode 71 and the cellular portions (specifically, the liquid in the first chamber 101 of the measurement chamber 100). A resistance Rr2 corresponds to the electrical resistance of the liquid between the lower reference electrode 73 and the cellular portions (specifically, the liquid in the second chamber 102 of the measurement chamber 100).

Voltage is applied between the upper working electrode 61 and the lower working electrode 63 by the power supply device 91, and voltage between the upper reference electrode 71 and the lower reference electrode 73 is measured by the voltmeter 92. The precise voltage value between the upper working electrode 61 and the lower working electrode 63 is calculated from the measured voltage value by a computer not shown. The resistance Rm of the cellular portions is calculated from the calculated voltage value. The electrical resistance of the cellular tissue 9 is determined by subtracting the resistance value of the porous membrane 30 from the resistance Rm of the cellular portions. The resistance value of the porous membrane 30 is determined by measuring the electrical resistance in the absence of the cellular tissue 9.

When voltage is applied between the upper working electrode 61 and the lower working electrode 63, the oxidation and reduction reactions of the liquid occur on surfaces of the upper and lower working electrodes 61 and 63 to form an electric double layer in some cases. When the electric double layer is formed, there is apprehension that the voltage applied between the upper working electrode 61 and the lower working electrode 63 differs from an output voltage caused by the power supply device 91. On the other hand, the upper and lower reference electrodes 71 and 73 are disposed in the vicinity of the upper and lower working electrodes 61 and 63 in the measurement chamber 100. Thus, the voltage between the upper working electrode 61 and the lower working electrode 63 is approximately acquired from the measured voltage value between the upper reference electrode 71 and the lower reference electrode 73. This allows the accurate measurement of the resistance Rm of the cellular portions.

### <Current Density Distribution>

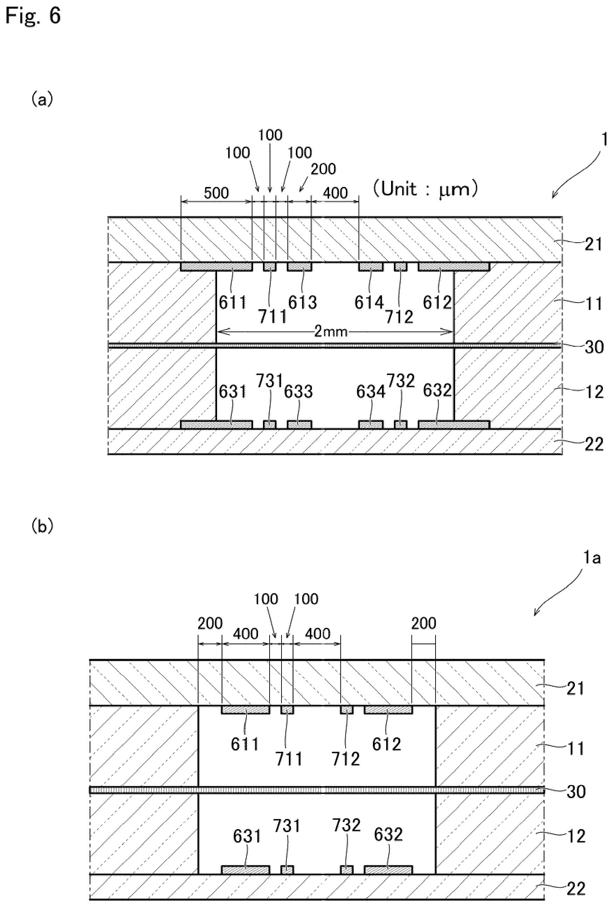

Next, simulation results of current density will be described. Fig. 6 is a view showing sectional structures of channel devices 1 and 1a used for simulation. Fig. 6(a) is a sectional view of the channel device 1 according to the embodiment, and Fig. 6(b) is a sectional view of the channel device 1a according to a comparative example. Fig. 6 illustrates the sizes of respective electrode portions or distances between the electrode portions.

In the channel device 1a according to the comparative example, as shown in Fig. 6(b), the first and second upper working portions 611 and 612 of the upper working electrode 61 and the first and second lower working portions 631 and 632 of the lower working electrode 63 are spaced inwardly of the measurement chamber 100 apart from the widthwise edges of the measurement chamber 100. In the channel device 1a, the third and fourth upper working portions 613 and 614 of the upper working electrode 61 and the third and fourth lower working portions 633 and 634 of the lower working electrode 63 are omitted.

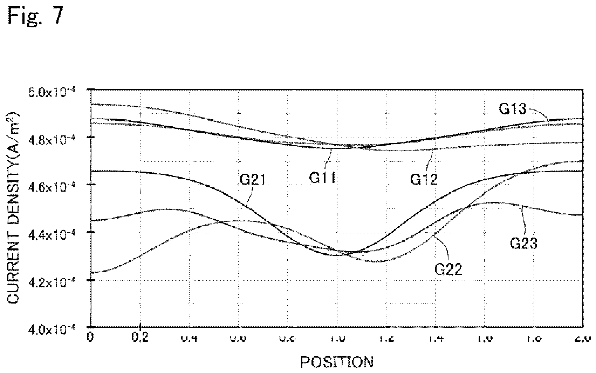

Fig. 7 is a graph showing simulation results of a current density distribution. In Fig. 7, the abscissa represents the position in the width direction of the measurement chamber 100, and the ordinate represents the current density distribution. In Fig. 7, curves G11 to G13 represent simulation results of the channel device 1 according to the embodiment, and curves G21 to G23 represent simulation results of the channel device 1 {1a} according to the comparative example.

The curves G11 and G21 represent simulation results obtained in the case where the first lid member 21 and the second lid member 22 mounted to the measurement chamber 100 are not displaced. The curves G12 and G22 represent simulation results obtained in the case where the first lid member 21 and the second lid member 22 mounted to the measurement chamber 100 are displaced toward the first side in the width direction with respect to the measurement chamber 100. The curves G13 and G23 represent simulation results obtained in the case where the first lid member 21 and the second lid member 22 mounted to the measurement chamber 100 are displaced toward the first side and toward the second side, respectively, in the width direction with respect to the measurement chamber 100.

In the case of the channel device 1a according to the comparative example, the current density distribution is fluctuated significantly by the displacement of the first lid member 21 and the second lid member 22 in the width direction, as represented by the curves G21 to G23. In particular, when the first lid member 21 and the second lid member 22 are displaced in the same direction as represented by the curve G22, the difference between the current density at a second edge (position "0") of the measurement chamber 100 as seen in the width direction and the current density at a first edge (position "2.0") of the measurement chamber 100 as seen in the width direction is relatively large. In the case of the channel device 1, on the other hand, the fluctuations in current density distribution is sufficiently small as compared to that of the channel device 1a when the first lid member 21 and the second lid member 22 are displaced in the width direction as represented by the curves G11 to G13.

If the current density distribution is non-uniform, there is apprehension that the electrical properties of part of a cell layer {the cellular portions} where the current density is high strongly influences the measurement results. In the channel device 1, the fluctuations in current density distribution is small even if the position of the first lid member 21 or the second lid member 22 is displaced. This suppresses variations in performance of the channel device 1 due to the assembly accuracy.

The channel device 1 is capable of applying voltage to the measurement chamber 100 uniformly throughout the width direction because the channel device 1 includes the third upper working portion 613, the fourth upper working portion 614, the third lower working portion 633, and the fourth lower working portion 634. Thus, the current densities of the channel device 1 (the curves G11 to G13) are more uniform than those of the channel device 1a (the curves G21 to G23). The channel device 1 of the present embodiment is capable of providing the uniform current density in this manner to suppress the strong influence of only part of the cellular portions on the measurement results.

### <Frequency Characteristics>

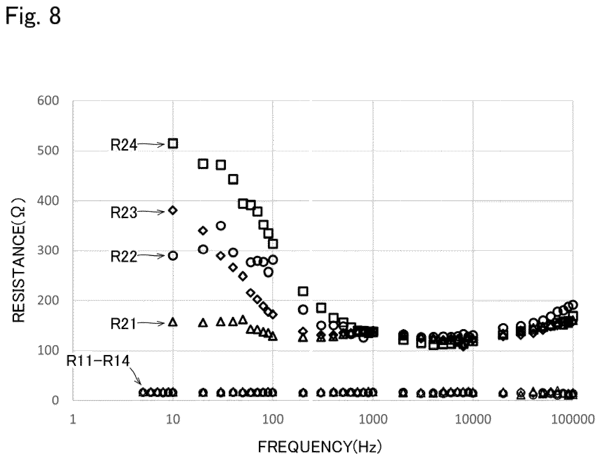

Fig. 8 is a graph showing frequency characteristics of the channel device 1 according to the embodiment and the channel device 1a according to the comparative example. In Fig. 8, the abscissa represents frequency (Hz), and the ordinate represents resistance (Ω). Fig. 8 shows results obtained by introducing the liquid (the culture medium) into the measurement chamber 100 of the channel device 1 or the channel device 1a, sweeping the frequency of the applied voltage, and measuring the resistance. Measurement results R11 to R14 for four channel devices 1 and measurement results R21 to R24 for four channel devices 1a are shown in Fig. 8.

As shown in Fig. 8, the measurement results R11 to R14 of the channel devices 1 have smaller variations in measurement values among the devices in each frequency region than the measurement results R21 to R24 of the channel devices 1a according to the comparative example. Especially in a low frequency region (around 10 Hz) used in TEER measurement, variations in measurement values among the four channel devices 1 are smaller than variations in measurement values among the four channel devices 1a.

Impedance measurement provides an estimation of the resistance and capacitance components of the cellular tissue 9 from the measurement results over a wide frequency region. In the case of the channel devices 1, variations in measurement values among the devices are suppressed over the entire frequency region. Thus, the measurement accuracy of the impedance measurement is improved by employing the channel devices 1.

### <2. Modifications>

While the embodiment according to the present invention has been described hereinabove, the present invention is not limited to the aforementioned embodiment, but various modifications may be made..

For example, it is not essential that the upper working electrode 61, the lower working electrode 63, the upper reference electrode 71, and the lower reference electrode 73 longitudinally traverse the measurement chamber 100.

It is not essential that the upper working electrode 61 includes the third upper working portion 613 or the fourth upper working portion 614. It is not essential that the lower working electrode 63 includes the third lower working portion 633 and the fourth lower working portion 634.

The upper working electrode 61 may further include an additional electrode portion different from the third and fourth upper working portions 613 and 614 between the first upper working portion 611 and the second upper working portion 612 as seen in the width direction. The lower working electrode 63 may further include an additional electrode portion between the first lower working portion 631 and the second lower working portion 632 as seen in the width direction.

### Reference Signs List

- 1: Channel device
- 10: Intermediate member
- 100: Measurement chamber
- 101: First chamber
- 102: Second chamber
- 10H: Through hole
- 11: First intermediate member
- 11H: First through hole
- 12: Second intermediate member
- 12H: Second through hole
- 1a: Channel device
- 21: First lid member
- 22: Second lid member
- 30: Porous membrane
- 43: First through hole
- 45: Second through hole
- 51: First channel
- 53: Second channel
- 61: Upper working electrode (first-side working electrode)
- 611: First upper working portion (first first-side working portion)
- 612: Second upper working portion (second first-side working portion)
- 613: Third upper working portion (third first-side working portion)
- 614: Fourth upper working portion (fourth first-side working portion)
- 63: Lower working electrode (second-side working electrode)
- 631: First lower working portion (first second-side working portion)
- 632: Second lower working portion (second second-side working portion)
- 633: Third lower working portion (third second-side working portion)
- 634: Fourth lower working portion (fourth second-side working portion)
- 71: Upper reference electrode (first-side reference electrode)
- 711: First upper reference portion (first first-side reference portion)
- 712: Second upper reference portion (second first-side reference portion)
- 73: Lower reference electrode (second-side reference electrode)
- 731: First lower reference portion (first second-side reference portion)
- 732: Second lower reference portion (second second-side reference portion)
- 9: Cellular tissue

## Claims

1. A channel device (1, 1a) for application to measurement of electrical resistance of cellular tissue (9), comprising:
an intermediate member (10) including a measurement chamber (100) formed by a through hole passing therethrough in a first direction;
a first lid member (21) positioned on a surface of said intermediate member (10) which is on a first side as seen in said first direction and closing an opening at a first edge of said measurement chamber (100) as seen in said first direction;
a second lid member (22) positioned on a surface of said intermediate member (10) which is on a second side as seen in said first direction and closing an opening at a second edge of said measurement chamber (100) as seen in said first direction;
a porous membrane (30) positioned in said measurement chamber (100) and partitioning said measurement chamber (100) into a first chamber (101) on the first side and a second chamber (102) on the second side as seen in said first direction, said porous membrane (30) being permeable to liquid;
a first-side working electrode (61) and a first-side reference electrode (71) both positioned on a surface of said first lid member (21) which is on the second side as seen in said first direction and overlapping said measurement chamber (100) as seen in said first direction; and
a second-side working electrode (63) and a second-side reference electrode (73) both positioned on a surface of said second lid member (22) which is on the first side as seen in said first direction and overlapping said measurement chamber (100) as seen in said first direction,
wherein said first-side working electrode (61) includes
a first first-side working portion (611), and
a second first-side working portion (612) spaced toward a first side in a second direction intersecting said first direction apart from said first first-side working portion (611),
**characterized in that**
a dimension from an edge of said first first-side working portion (611) which is on a second side as seen in said second direction to an edge of said second first-side working portion (612) which is on the first side as seen in said second direction is greater than the width of said measurement chamber (100) as measured in said second direction, and
wherein a distance between said first first-side working portion (611) and said second first-side working portion (612) as measured in said second direction is smaller than the width of said measurement chamber (100).

2. The channel device (1, 1a) according to Claim 1,
wherein said first-side reference electrode (71) includes
a first first-side reference portion (711), and
a second first-side reference portion (712) spaced toward the first side in said second direction apart from said first first-side reference portion (711), and
wherein said first first-side reference portion (711) and said second first-side reference portion (712) are positioned between said first first-side working portion (611) and said second first-side working portion (612) as seen in said second direction.

3. The channel device (1, 1a) according to Claim 2,
wherein said first-side working electrode (61) further includes a third first-side working portion (613) positioned between said first first-side reference portion (711) and said second first-side reference portion (712) as seen in said second direction.

4. The channel device (1, 1a) according to Claim 3,
wherein the width of said first first-side working portion (611) as measured in said second direction is greater than the width of said third first-side working portion (613) as measured in said second direction.

5. The channel device (1, 1a) according to Claim 3 or 4,
wherein said first-side working electrode (61) further includes a fourth first-side working portion (614) positioned between said first first-side reference portion (711) and said second first-side reference portion (712) as seen in said second direction and spaced toward the first side in said second direction apart from said third first-side working portion (613).

6. The channel device (1, 1a) according to Claim 5,
wherein said second-side working electrode (63) includes
a first second-side working portion (631), and
a second second-side working portion (632) spaced toward the first side in said second direction apart from said first second-side working portion (631),
wherein said first second-side working portion (631) is in opposed relation to said first first-side working portion (611) as seen in said first direction,
wherein said second second-side working portion (632) is in opposed relation to said second first-side working portion (612) as seen in said first direction,
wherein a dimension from an edge of said first second-side working portion (631) which is on the second side as seen in said second direction to an edge of said second second-side working portion (632) which is on the first side as seen in said second direction is greater than the width of said measurement chamber (100) as measured in said second direction, and
wherein a distance between said first second-side working portion (631) and said second second-side working portion (632) as measured in said second direction is smaller than the width of said measurement chamber (100) as measured in said second direction.

7. The channel device (1, 1a) according to Claim 6,
wherein said second-side reference electrode (73) includes
a first second-side reference portion (731), and
a second second-side reference portion (732) spaced toward the first side in said second direction apart from said first second-side reference portion (731), and
wherein said first second-side reference portion (731) and said second second-side reference portion (732) are positioned between said first second-side working portion (631) and said second second-side working portion (632) as seen in said second direction.

8. The channel device (1, 1a) according to Claim 7,
wherein said second-side working electrode (63) further includes a third second-side working portion (633) positioned between said first second-side reference portion (731) and said second second-side reference portion (732) as seen in said second direction.

9. The channel device (1, 1a) according to Claim 8,
wherein said second-side working electrode (63) further includes a fourth second-side working portion (634) positioned between said first second-side reference portion (731) and said second second-side reference portion (732) as seen in said second direction and spaced toward the first side in said second direction apart from said third second-side working portion (633).

10. The channel device (1, 1a) according to any one of Claims 1 to 9,
wherein said intermediate member (10) includes
a first intermediate member (11), and
a second intermediate member (12) positioned on the first side of said first intermediate member (11) as seen in said first direction,
wherein said first intermediate member (11) includes said first chamber (101),
wherein said second intermediate member (12) includes said second chamber (102), and
wherein said porous membrane (30) is positioned between said first intermediate member (11) and said second intermediate member (12) as seen in said first direction.

11. The channel device (1, 1a) according to any one of Claims 1 to 10,
wherein said intermediate member (10) includes
a first channel (51) in communication with said first chamber (101), and
a second channel (53) in communication with said second chamber (102).

12. The channel device (1, 1a) according to Claim 11,
wherein said first lid member (21) includes
a first through hole (43) in communication with said first channel (51), and
a second through hole (45) in communication with said second channel (53).

13. The channel device (1, 1a) according to any one of Claims 1 to 12,
wherein said measurement chamber (100), said first first-side working portion (611), and said second first-side working portion (612) extend in a third direction intersecting said first and second directions.

14. The channel device (1, 1a) according to Claim 13,
wherein said first first-side working portion (611) and said second first-side working portion (612) extend from a position spaced toward a first side in said third direction apart from said measurement chamber (100) to a position spaced toward a second side in said third direction apart from said measurement chamber (100).

## Patentansprüche

1. Kanalvorrichtung (1, 1a) zur Anwendung bei der Messung des elektrischen Widerstands von Zellgewebe (9), umfassend:
ein Zwischenelement (10), das eine Messkammer (100) umfasst, die durch ein Durchgangsloch gebildet ist, das in einer ersten Richtung dort hindurch verläuft;
ein erstes Deckelelement (21), das auf einer Oberfläche des Zwischenelements (10) positioniert ist, die sich in der ersten Richtung gesehen auf einer ersten Seite befindet, und das eine Öffnung an einer ersten Kante der Messkammer (100) in der ersten Richtung gesehen verschließt;
ein zweites Deckelelement (22), das auf einer Oberfläche des Zwischenelements (10) positioniert ist, die sich in der ersten Richtung gesehen auf einer zweiten Seite befindet, und das eine Öffnung an einer zweiten Kante der Messkammer (100) in der ersten Richtung gesehen verschließt;
eine poröse Membran (30), die in der Messkammer (100) positioniert ist und die Messkammer (100) in eine erste Kammer (101) auf der ersten Seite und eine zweite Kammer (102) auf der zweiten Seite in der ersten Richtung gesehen unterteilt, wobei die poröse Membran (30) für Flüssigkeit durchlässig ist;
eine Arbeitselektrode (61) auf der ersten Seite und eine Referenzelektrode (71) auf der ersten Seite, die beide auf einer Oberfläche des ersten Deckelelements (21) positioniert sind, die sich in der ersten Richtung gesehen auf der zweiten Seite befindet, und die die Messkammer (100) in der ersten Richtung gesehen überlappen; und
eine Arbeitselektrode (63) auf der zweiten Seite und eine Referenzelektrode (73) auf der zweiten Seite, die beide auf einer Oberfläche des zweiten Deckelelements (22) positioniert sind, die sich in der ersten Richtung gesehen auf der ersten Seite befindet, und die die Messkammer (100) in der ersten Richtung gesehen überlappen;
wobei die Arbeitselektrode (61) auf der ersten Seite umfasst:
einen ersten Arbeitsabschnitt (611) auf der ersten Seite, und
einen zweiten Arbeitsabschnitt (612) auf der ersten Seite, der in Richtung einer ersten Seite in einer zweiten Richtung, die die erste Richtung schneidet, von dem ersten Arbeitsabschnitt (611) auf der ersten Seite beabstandet ist,
**dadurch gekennzeichnet, dass** eine Abmessung von einer Kante des ersten Arbeitsabschnitts (611) auf der ersten Seite, die sich in der zweiten Richtung gesehen auf einer zweiten Seite befindet, zu einer Kante des zweiten Arbeitsabschnitts (612) auf der ersten Seite, die sich in der zweiten Richtung gesehen auf der ersten Seite befindet, größer ist als die Breite der Messkammer (100), gemessen in der zweiten Richtung, und
wobei ein Abstand zwischen dem ersten Arbeitsabschnitt (611) auf der ersten Seite und dem zweiten Arbeitsabschnitt (612) auf der ersten Seite, gemessen in der zweiten Richtung, kleiner ist als die Breite der Messkammer (100).

2. Kanalvorrichtung (1, 1a) nach Anspruch 1,
wobei die Referenzelektrode (71) auf der ersten Seite umfasst:
einen ersten Referenzabschnitt (711) auf der ersten Seite, und
einen zweiten Referenzabschnitt (712) auf der ersten Seite, der in Richtung der ersten Seite in der zweiten Richtung von dem ersten Referenzabschnitt (711) auf der ersten Seite beabstandet ist, und
wobei der erste Referenzabschnitt (711) auf der ersten Seite und der zweite Referenzabschnitt (712) auf der ersten Seite zwischen dem ersten Arbeitsabschnitt (611) auf der ersten Seite und dem zweiten Arbeitsabschnitt (612) auf der ersten Seite, in der zweiten Richtung gesehen, positioniert sind.

3. Kanalvorrichtung (1, 1a) nach Anspruch 2,
wobei die Arbeitselektrode (61) auf der ersten Seite ferner einen dritten Arbeitsabschnitt (613) auf der ersten Seite umfasst, der zwischen dem ersten Referenzabschnitt (711) auf der ersten Seite und dem zweiten Referenzabschnitt (712) auf der ersten Seite, in der zweiten Richtung gesehen, positioniert ist.

4. Kanalvorrichtung (1, 1a) nach Anspruch 3,
wobei die Breite des ersten Arbeitsabschnitts (611) auf der ersten Seite, gemessen in der zweiten Richtung, größer ist als die Breite des dritten Arbeitsabschnitts (613) auf der ersten Seite, gemessen in der zweiten Richtung.

5. Kanalvorrichtung (1, 1a) nach Anspruch 3 oder 4,
wobei die Arbeitselektrode (61) auf der ersten Seite ferner einen vierten Arbeitsabschnitt (614) auf der ersten Seite umfasst, der zwischen dem ersten Referenzabschnitt (711) auf der ersten Seite und dem zweiten Referenzabschnitt (712) auf der ersten Seite, in der zweiten Richtung gesehen, positioniert ist und in Richtung der ersten Seite in der zweiten Richtung von dem dritten Arbeitsabschnitt (613) auf der ersten Seite beabstandet ist.

6. Kanalvorrichtung (1, 1a) nach Anspruch 5,
wobei die Arbeitselektrode (63) auf der zweiten Seite umfasst:
einen ersten Arbeitsabschnitt (631) auf der zweiten Seite, und
einen zweiten Arbeitsabschnitt (632) auf der zweiten Seite, der in Richtung der ersten Seite in der zweiten Richtung von dem ersten Arbeitsabschnitt (631) auf der zweiten Seite beabstandet ist,
wobei der erste Arbeitsabschnitt (631) auf der zweiten Seite in entgegengesetzter Beziehung zu dem ersten Arbeitsabschnitt (611) auf der ersten Seite, in der ersten Richtung gesehen, steht,
wobei der zweite Arbeitsabschnitt (632) auf der zweiten Seite in entgegengesetzter Beziehung zu dem zweiten Arbeitsabschnitt (612) auf der ersten Seite, in der ersten Richtung gesehen, steht,
wobei eine Abmessung von einer Kante des ersten Arbeitsabschnitts (631) auf der zweiten Seite, die sich in der zweiten Richtung gesehen auf der zweiten Seite befindet, zu einer Kante des zweiten Arbeitsabschnitts (632) auf der zweiten Seite, die sich in der zweiten Richtung gesehen auf der ersten Seite befindet, größer ist als die Breite der Messkammer (100), gemessen in der zweiten Richtung, und
wobei ein Abstand zwischen dem ersten Arbeitsabschnitt (631) auf der zweiten Seite und dem zweiten Arbeitsabschnitt (632) auf der zweiten Seite, gemessen in der zweiten Richtung, kleiner ist als die Breite der Messkammer (100), gemessen in der zweiten Richtung.

7. Kanalvorrichtung (1, 1a) nach Anspruch 6,
wobei die Referenzelektrode (73) auf der zweiten Seite umfasst:
einen ersten Referenzabschnitt (731) auf der zweiten Seite, und
einen zweiten Referenzabschnitt (732) auf der zweiten Seite, der in Richtung der ersten Seite in der zweiten Richtung von dem ersten Referenzabschnitt (731) auf der zweiten Seite beabstandet ist, und
wobei der erste Referenzabschnitt (731) auf der zweiten Seite und der zweite Referenzabschnitt (732) auf der zweiten Seite zwischen dem ersten Arbeitsabschnitt (631) auf der zweiten Seite und dem zweiten Arbeitsabschnitt (632) auf der zweiten Seite, in der zweiten Richtung gesehen, positioniert sind.

8. Kanalvorrichtung (1, 1a) nach Anspruch 7,
wobei die Arbeitselektrode (63) auf der zweiten Seite ferner einen dritten Arbeitsabschnitt (633) auf der zweiten Seite umfasst, der zwischen dem ersten Referenzabschnitt (731) auf der zweiten Seite und dem zweiten Referenzabschnitt (732) auf der zweiten Seite, in der zweiten Richtung gesehen, positioniert ist.

9. Kanalvorrichtung (1, 1a) nach Anspruch 8,
wobei die Arbeitselektrode (63) auf der zweiten Seite ferner einen vierten Arbeitsabschnitt (634) auf der zweiten Seite umfasst, der zwischen dem ersten Referenzabschnitt (731) auf der zweiten Seite und dem zweiten Referenzabschnitt (732) auf der zweiten Seite, in der zweiten Richtung gesehen, positioniert ist und in Richtung der ersten Seite in der zweiten Richtung von dem dritten Arbeitsabschnitt (633) auf der zweiten Seite beabstandet ist.

10. Kanalvorrichtung (1, 1a) nach einem der Ansprüche 1 bis 9,
wobei das Zwischenelement (10) umfasst
ein erstes Zwischenelement (11), und
ein zweites Zwischenelement (12), das auf der ersten Seite des ersten Zwischenelements (11), in der ersten Richtung gesehen, positioniert ist,
wobei das erste Zwischenelement (11) die erste Kammer (101) umfasst,
wobei das zweite Zwischenelement (12) die zweite Kammer (102) umfasst, und
wobei die poröse Membran (30) zwischen dem ersten Zwischenelement (11) und dem zweiten Zwischenelement (12), in der ersten Richtung gesehen, positioniert ist.

11. Kanalvorrichtung (1, 1a) nach einem der Ansprüche 1 bis 10,
wobei das Zwischenelement (10) umfasst
einen ersten Kanal (51) in Kommunikation mit der ersten Kammer (101), und
einen zweiten Kanal (53) in Kommunikation mit der zweiten Kammer (102).

12. Kanalvorrichtung (1, 1a) nach Anspruch 11,
wobei das erste Deckelelement (21) umfasst
ein erstes Durchgangsloch (43) in Kommunikation mit dem ersten Kanal (51), und
ein zweites Durchgangsloch (45) in Kommunikation mit dem zweiten Kanal (53).

13. Kanalvorrichtung (1, 1a) nach einem der Ansprüche 1 bis 12,
wobei sich die Messkammer (100), der erste Arbeitsabschnitt (611) auf der ersten Seite und der zweite Arbeitsabschnitt (612) auf der ersten Seite in einer dritten Richtung erstrecken, die die erste und die zweite Richtung schneidet.

14. Kanalvorrichtung (1, 1a) nach Anspruch 13,
wobei sich der erste Arbeitsabschnitt (611) auf der ersten Seite und der zweite Arbeitsabschnitt (612) auf der ersten Seite von einer Position, die in Richtung einer ersten Seite in der dritten Richtung von der Messkammer (100) beabstandet ist, zu einer Position erstrecken, die in Richtung einer zweiten Seite in der dritten Richtung von der Messkammer (100) beabstandet ist.

## Revendications

1. Dispositif de canal (1, 1a) destiné à une application de mesure de résistance électrique de tissu cellulaire (9), comprenant :
un élément intermédiaire (10) incluant une chambre de mesure (100) formée par un trou traversant y passant dans une première direction ;
un premier élément du couvercle (21) positionné sur une surface dudit élément intermédiaire (10) qui est sur un premier côté, vu dans ladite première direction et fermant une ouverture à un premier bord de ladite chambre de mesure (100), vu dans ladite première direction ;
un deuxième élément du couvercle (22) positionné sur une surface dudit élément intermédiaire (10) qui est sur un deuxième côté, vu dans ladite première direction et fermant une ouverture à un deuxième bord de ladite chambre de mesure (100), vu dans ladite première direction ;
une membrane poreuse (30) positionnée dans ladite chambre de mesure (100) et divisant ladite chambre de mesure (100) en une première chambre (101) sur le premier côté et une deuxième chambre (102) sur le deuxième côté, vu dans ladite première direction, ladite membrane poreuse (30) étant perméable à un liquide ;
une électrode de travail de premier côté (61) et une électrode de référence de premier côté (71) toutes deux positionnées sur une surface dudit premier élément du couvercle (21) qui est sur le deuxième côté, vu dans ladite première direction et chevauchant ladite chambre de mesure (100), vu dans ladite première direction ; et
une électrode de travail de deuxième côté (63) et une électrode de référence de deuxième côté (73) toutes deux positionnées sur une surface dudit deuxième élément du couvercle (22) qui est sur le premier côté, vu dans ladite première direction et chevauchant ladite chambre de mesure (100), vu dans ladite première direction,
dans lequel ladite électrode de travail de premier côté (61) inclut
une première partie de travail de premier côté (611), et
une deuxième partie de travail de premier côté (612) espacée vers un premier côté dans une deuxième direction qui coupe ladite première direction écartée de ladite première partie de travail de premier côté (611),
**caractérisé en ce que**
une dimension depuis un bord de ladite première partie de travail de premier côté (611) qui est sur un deuxième côté, vu dans ladite deuxième direction, à un bord de ladite deuxième partie de travail de premier côté (612) qui est sur le premier côté, vu dans ladite deuxième direction, est supérieure à la largeur de ladite chambre de mesure (100), mesurée dans ladite deuxième direction, et
dans lequel une distance entre ladite première partie de travail de premier côté (611) et ladite deuxième partie de travail de premier côté (612), mesurée dans ladite deuxième direction, est inférieure à la largeur de ladite chambre de mesure (100).

2. Dispositif de canal (1, 1a) selon la revendication 1,
dans lequel ladite électrode de référence de premier côté (71) inclut
une première partie de référence de premier côté (711), et
une deuxième partie de référence de premier côté (712) espacée vers le premier côté dans ladite deuxième direction écartée de ladite première partie de référence de premier côté (711), et
dans lequel ladite première partie de référence de premier côté (711) et ladite deuxième partie de référence de premier côté (712) sont positionnées entre ladite première partie de travail de premier côté (611) et ladite deuxième partie de travail de premier côté (612), vu dans ladite deuxième direction.

3. Dispositif de canal (1, 1a) selon la revendication 2,
dans lequel ladite électrode de travail de premier côté (61) inclut en outre une troisième partie de travail de premier côté (613) positionnée entre ladite première partie de référence de premier côté (711) et ladite deuxième partie de référence de premier côté (712), vu dans ladite deuxième direction.

4. Dispositif de canal (1, 1a) selon la revendication 3,
dans lequel la largeur de ladite première partie de travail de premier côté (611), mesurée dans ladite deuxième direction, est supérieure à la largeur de ladite troisième partie de travail de premier côté (613), mesurée dans ladite deuxième direction.

5. Dispositif de canal (1, 1a) selon la revendication 3 ou 4,
dans lequel ladite électrode de travail de premier côté (61) inclut en outre une quatrième partie de travail de premier côté (614) positionnée entre ladite première partie de référence de premier côté (711) et ladite deuxième partie de référence de premier côté (712), vu dans ladite deuxième direction, et espacée vers le premier côté dans ladite deuxième direction écartée de ladite troisième partie de travail de premier côté (613).

6. Dispositif de canal (1, 1a) selon la revendication 5,
dans lequel ladite électrode de travail de deuxième côté (63) inclut
une première partie de travail de deuxième côté (631), et
une deuxième partie de travail de deuxième côté (632) espacée vers le premier côté dans ladite deuxième direction écartée de ladite première partie de travail de deuxième côté (631),
dans lequel ladite première partie de travail de deuxième côté (631) est dans une position opposée à ladite première partie de travail de premier côté (611), vu dans ladite première direction,
dans lequel ladite deuxième partie de travail de deuxième côté (632) est dans une position opposée à ladite deuxième partie de travail de premier côté (612), vu dans ladite première direction,
dans lequel une dimension depuis un bord de ladite première partie de travail de deuxième côté (631) qui est sur le deuxième côté, vu dans ladite deuxième direction, à un bord de ladite deuxième partie de travail de deuxième côté (632) qui est sur le premier côté, vu dans ladite deuxième direction, est supérieure à la largeur de ladite chambre de mesure (100), mesurée dans ladite deuxième direction, et
dans lequel une distance entre ladite première partie de travail de deuxième côté (631) et ladite deuxième partie de travail de deuxième côté (632), mesurée dans ladite deuxième direction, est inférieure à la largeur de ladite chambre de mesure (100), mesurée dans ladite deuxième direction.

7. Dispositif de canal (1, 1a) selon la revendication 6,
dans lequel ladite électrode de référence de deuxième côté (73) inclut
une première partie de référence de deuxième côté (731), et
une deuxième partie de référence de deuxième côté (732) espacée vers le premier côté dans ladite deuxième direction écartée de ladite première partie de référence de deuxième côté (731), et
dans lequel ladite première partie de référence de deuxième côté (731) et ladite deuxième partie de référence de deuxième côté (732) sont positionnées entre ladite première partie de travail de deuxième côté (631) et ladite deuxième partie de travail de deuxième côté (632), vu dans ladite deuxième direction.

8. Dispositif de canal (1, 1a) selon la revendication 7,
dans lequel ladite électrode de travail de deuxième côté (63) inclut en outre une troisième partie de travail de deuxième côté (633) positionnée entre ladite première partie de référence de deuxième côté (731) et ladite deuxième partie de référence de deuxième côté (732), vu dans ladite deuxième direction.

9. Dispositif de canal (1, 1a) selon la revendication 8,
dans lequel ladite électrode de travail de deuxième côté (63) inclut en outre une quatrième partie de travail de deuxième côté (634) positionnée entre ladite première partie de référence de deuxième côté (731) et ladite deuxième partie de référence de deuxième côté (732), vu dans ladite deuxième direction, et espacée vers le premier côté dans ladite deuxième direction écartée de ladite troisième partie de travail de deuxième côté (633).

10. Dispositif de canal (1, 1a) selon l'une quelconque des revendications 1 à 9,
dans lequel ledit élément intermédiaire (10) inclut
un premier élément intermédiaire (11), et
un deuxième élément intermédiaire (12) positionné sur le premier côté dudit premier élément intermédiaire (11), vu dans ladite première direction,
dans lequel ledit premier élément intermédiaire (11) inclut ladite première chambre (101),
dans lequel ledit deuxième élément intermédiaire (12) inclut ladite deuxième chambre (102), et
dans lequel ladite membrane poreuse (30) est positionnée entre ledit premier élément intermédiaire (11) et ledit deuxième élément intermédiaire (12), vu dans ladite première direction.

11. Dispositif de canal (1, 1a) selon l'une quelconque des revendications 1 à 10,
dans lequel ledit élément intermédiaire (10) inclut
un premier canal (51) en communication avec ladite première chambre (101), et
un deuxième canal (53) en communication avec ladite deuxième chambre (102).

12. Dispositif de canal (1, 1a) selon la revendication 11,
dans lequel ledit premier élément du couvercle (21) inclut
un premier trou traversant (43) en communication avec ledit premier canal (51), et
un deuxième trou traversant (45) en communication avec ledit deuxième canal (53).

13. Dispositif de canal (1, 1a) selon l'une quelconque des revendications 1 à 12,
dans lequel ladite chambre de mesure (100), ladite première partie de travail de premier côté (611) et ladite deuxième partie de travail de premier côté (612) s'étendent dans une troisième direction qui coupe lesdites première et deuxième directions.

14. Dispositif de canal (1, 1a) selon la revendication 13,
dans lequel ladite première partie de travail de premier côté (611) et ladite deuxième partie de travail de premier côté (612) s'étendent depuis une position espacée vers un premier côté dans ladite troisième direction écartée de ladite chambre de mesure (100) à une position espacée vers un deuxième côté dans ladite troisième direction écartée de ladite chambre de mesure (100).
